# EUROPEAN PATENT APPLICATION

(11) **EP 4 327 805 A1**
(43) Date of publication of application: **28.02.2024**
(21) Application number: 22794795.9
(22) Date of filing: 24.04.2022
(51) Int. Cl.: A61K 9/16, A61K 49/00

(54) **13C METHACETIN GRANULE, AND PREPARATION METHOD THEREFOR AND USE THEREOF**

(30) Priority: 30.04.2021 CN 202110484540
(71) Applicant: Beijing Richen-Force Science & Technology Co., Ltd, Beijing 100015 (CN); Jiangsu Richen-Bases Bio & Sci Co., Ltd., Taizhou, Jiangsu 225300 (CN)
(72) Inventor: GONG, Aihua, Beijing 100015 (CN)
(74) Representative: Zenz Patentanwälte Partnerschaft mbB
(86) International application number: PCT/CN2022/088678
(87) International publication number: WO 2022/228324

(57) **Abstract**

The present invention relates to the field of pharmaceutical preparations, in particular to a 13C-methacetin granule, and a preparation method therefor and the use thereof. Provided is the 13C-methacetin granule, comprising 13C-methacetin, mannitol, a co-solvent and a wetting agent. The 13C-methacetin granule has good stability, is beneficial for storage and the production of subsequent preparations, etc. The 13C-methacetin granule has good water solubility, can be rapidly dissolved in water during clinical use, and is convenient to take. Further provided is the use of the 13C-methacetin granule in the preparation of drugs, reagents or kits for disease diagnosis, evaluation of the liver reserve function and preoperative and postoperative evaluation of the liver. The provided preparation method of the 13C-methacetin granule has mild conditions, simple operation and convenient production control.

## Description

### Cross Reference to Related Applications

This application claims priority to Chinese Patent Application No. 202110484540.1, filed with the Chinese Patent Office on April 30, 2021, entitled "13C Methacetin granule, and Preparation Method Therefor and Use Thereof", the entirety of which is incorporated herein by reference.

### Technical Field

The present disclosure relates to the field of pharmaceutical formulations, and in particular to a 13C-methacetin granular formulation and a preparation method therefor and use thereof.

### Background Art

The liver is an important metabolic organ in a human body. Although there are many indicators of liver function tests currently used in clinical practice, there are few tests that can quantitatively reflect the hepatic reserve and compensatory capabilities in the early stage of liver cirrhosis. The 13C-methacetin breath test is a recently-emerging new method for detecting hepatic functional reserve. It is found through experiments that the 13C-methacetin breath test values can distinguish whether it is liver cirrhosis and are highly meaningful in clinical practice.

Here, 13C-methacetin has a chemical name of N-acetyl-p-anisidine (methoxy-13C) and has a structural formula as shown in formula I:

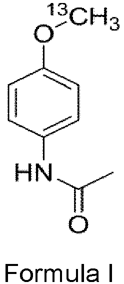

In the research of pharmaceutical formulations, more and more attention is paid to the dosage forms of products, because different dosage forms of drugs may have different degrees of influence on the stability, oral administration, and absorption of the drugs, etc. 13C-methacetin is used for diagnosis and needs to be rapidly absorbed in vivo, thus this product needs to be dissolved in water and orally administered as soon as possible during oral administration. It is of great significance to develop a dosage form of 13-methacetin with excellent solubility and stability, in order to ensure the quality stability of 13C-methacetin formulation products as well as the safety and effectiveness during oral administration and absorption thereof.

### Summary

In view of this, the present disclosure provides a 13C-methacetin granular formulation, and a preparation method therefor and use thereof. Compared with the traditional 13C-methacetin, the 13C-methacetin granular formulation described in the present disclosure has good stability and a uniform granule size and is advantageous in storage and in production of subsequent formulations. The method for preparing a 13C-methacetin granular formulation described in the present disclosure involves simple operations, has good repeatability, and achieves a high yield.

In order to achieve the above-mentioned objectives of the disclosure, the present disclosure provides the following technical solutions.

The present disclosure provides a 13C-methacetin granular formulation, comprising 13C-methacetin, mannitol, a cosolvent, and a wetting agent; wherein the cosolvent includes one or more of Tween 80, β-cyclodextrin, sodium hydroxide, sodium citrate, disodium hydrogen phosphate, sodium dihydrogen phosphate, polyethylene glycol 6000, polyethylene glycol 4000, polyethylene glycol 2000, and polyethylene glycol 1500; and the wetting agent includes one or both of water or an aqueous solution of ethanol.

In some specific embodiments of the present disclosure, a weight ratio of the 13C-methacetin to the cosolvent is 1: (0.1 to 100).

In some specific embodiments of the present disclosure, a weight ratio of a mixture of the 13C-methacetin, mannitol, and the cosolvent to the wetting agent is 100: (1 to 100), calculated as g/mL.

In some specific embodiments of the present disclosure, a weight ratio of a mixture of the 13C-methacetin and the cosolvent to the wetting agent is 100: (1 to 20), calculated as g/mL; the wetting agent is an aqueous solution of ethanol, and a volume ratio of ethanol to water in the aqueous solution of ethanol is 1:5 to 5:1.

In some specific embodiments of the present disclosure, a weight ratio of the 13C-methacetin to the mannitol is 1: (20 to 60).

The present disclosure further provides a method for preparing the 13C-methacetin granular formulation, wherein 13C-methacetin is pulverized and then mixed with mannitol and a cosolvent to obtain a mixture, and the mixture is mixed with a wetting agent and then granulated and dried to obtain a 13C-methacetin granular formulation.

In some specific embodiments of the present disclosure, the drying is performed at a temperature of 30 to 100 °C, and the drying is performed for a period of 1 to 8 h.

In some specific embodiments of the present disclosure, the drying is performed at a temperature of 45 °C, and the drying is performed for a period of 3 h.

The present disclosure further provides use of the 13C-methacetin granular formulation or of a 13C-methacetin granular formulation prepared by the preparation method in the preparation of drugs, reagents, or kits for diagnosis of a disease, evaluation of hepatic functional reserve, and preoperative and postoperative evaluation of the liver.

The present invent further provides a method for diagnosis of a disease, a method for evaluation of hepatic functional reserve, or a method for preoperative and postoperative evaluation of the liver, comprising: administering the 13C-methacetin granular formulation or a 13C-methacetin granular formulation prepared by the preparation method. The disease includes one or more of liver cirrhosis, primary biliary cirrhosis, simple steatosis, a stage of chronic liver disease, alcoholic liver disease, a grade or stage of liver fibrosis, non-alcoholic fatty liver disease, neonatal cholestasis, biliary atresia, intrahepatic inflammation, acute liver failure, and hepatocellular carcinoma; the evaluation of hepatic functional reserve includes one or more of assessment of liver function for a related disease, evaluation of liver injury from chemotherapy, prediction of death from chronic liver failure, and evaluation of liver detoxification capability; and the preoperative and postoperative evaluation includes one or more of evaluation of liver regeneration after liver resection, evaluation of the urgency of liver transplantation, evaluation of postoperative liver failure, and related preoperative and postoperative evaluation. In some specific embodiments of the present disclosure, the disease includes, but is not limited to, one or more of liver cirrhosis, primary biliary cirrhosis, simple steatosis, a stage of chronic liver disease, alcoholic liver disease, a grade or stage of liver fibrosis, non-alcoholic fatty liver disease, neonatal cholestasis, biliary atresia, intrahepatic inflammation, acute liver failure, and hepatocellular carcinoma;
the evaluation of hepatic functional reserve includes, but is not limited to, one or more of assessment of liver function for a related disease, evaluation of liver injury from chemotherapy, prediction of death from chronic liver failure, and evaluation of liver detoxification capability; and
the preoperative and postoperative evaluation includes, but is not limited to, one or more of evaluation of liver regeneration after liver resection, evaluation of the urgency of liver transplantation, evaluation of postoperative liver failure, and related preoperative and postoperative evaluation.

Compared with the prior art, the beneficial effects of the present disclosure include, but are not limited to, the following effects.
(1) The 13C-methacetin granular formulation described in the present disclosure has good stability and is advantageous in storage and in production of subsequent formulations, etc.
(2) The method for preparing a 13C-methacetin granular formulation described in the present disclosure involves mild conditions and simple operations and facilitates production control.
(3) The 13C-methacetin granular formulation described in the present disclosure has good water solubility, can be rapidly dissolved in water during clinical use, and is convenient for oral administration.
(4) The 13C-methacetin granular formulation described in the present disclosure can be used in the preparation of drugs, reagents, or kits for diagnosis of a disease, evaluation of hepatic functional reserve, and preoperative and postoperative evaluation of the liver, and has good stability.

The disease described in the present disclosure includes one or more of liver cirrhosis, primary biliary cirrhosis, simple steatosis, a stage of chronic liver disease, alcoholic liver disease, a grade or stage of liver fibrosis, non-alcoholic fatty liver disease, neonatal cholestasis, biliary atresia, intrahepatic inflammation, acute liver failure, and hepatocellular carcinoma.

The evaluation of hepatic functional reserve described in the present disclosure includes one or more of assessment of liver function for a related disease, evaluation of liver injury from chemotherapy, prediction of death from chronic liver failure, and evaluation of liver detoxification capability.

The preoperative and postoperative evaluation described in the present disclosure includes one or more of evaluation of liver regeneration after liver resection, evaluation of the urgency of liver transplantation, evaluation of postoperative liver failure, and related preoperative and postoperative evaluation.

### Brief Description of Drawings

The accompanying drawings required to be used in the description of the examples or the prior art are briefly described below, in order to illustrate the technical solutions in the examples of the present disclosure or in the prior art more clearly.

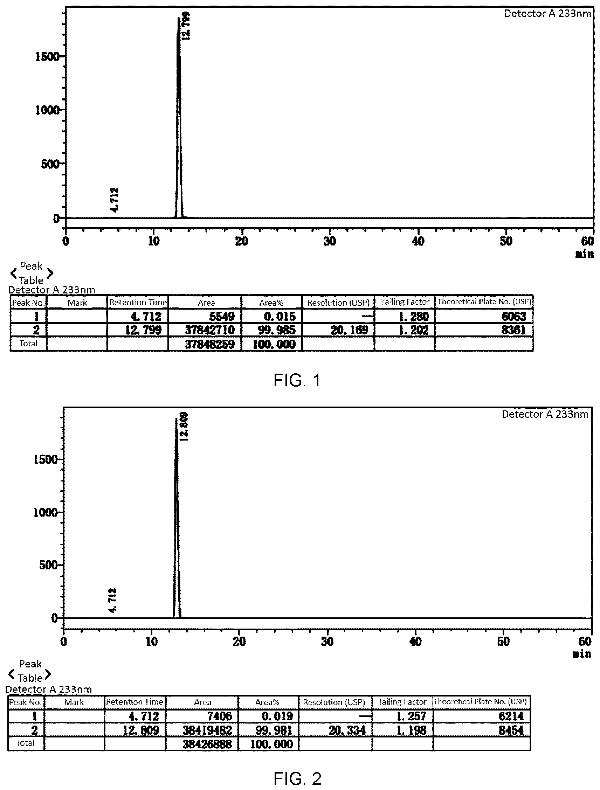

FIG. 1 shows a HPLC chromatogram of 13C-methacetin granules prepared in Example 1.
FIG. 2 shows a HPLC chromatogram of 13C-methacetin granules prepared in Example 2.

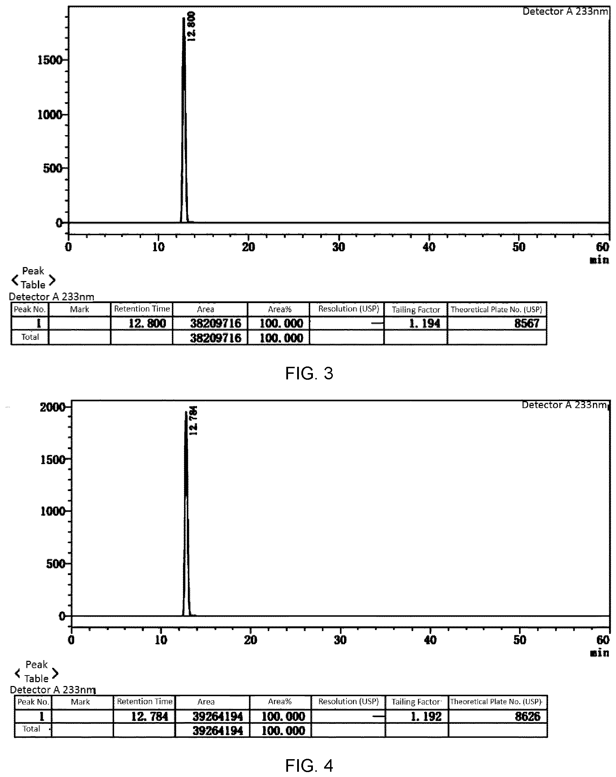

FIG. 3 shows a HPLC chromatogram of 13C-methacetin granules prepared in Example 3.
FIG. 4 shows a HPLC chromatogram of 13C-methacetin granules prepared in Example 4.

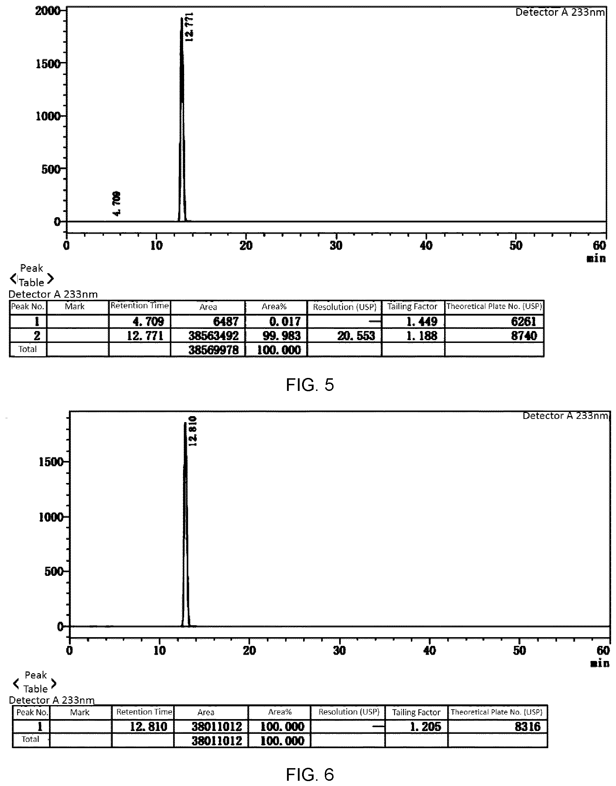

FIG. 5 shows a HPLC chromatogram of 13C-methacetin granules prepared in Example 5.
FIG. 6 shows a HPLC chromatogram of 13C-methacetin granules prepared in Example 6.

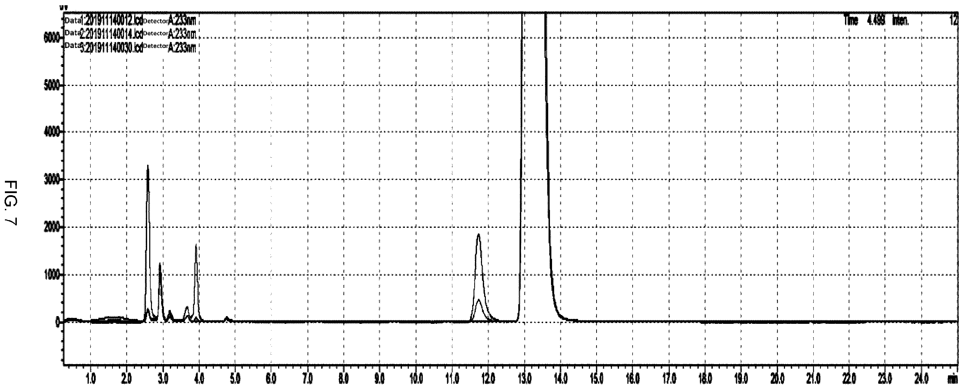

FIG. 7 shows results of comparison of water solubility and stability of the 13C-methacetin granules according to the present disclosure with those of a powdered formulation and another granular formulation.

### Detailed Description of Embodiments

The present disclosure discloses a 13C-methacetin granular formulation, a preparation method therefor, and use thereof, which can be implemented by those skilled in the art by appropriately modifying process parameters after learn from the disclosure herein. It should be particularly noted that all similar substitutions and variations are obvious to those skilled in the art, and they are deemed to be included in the present disclosure. Since the methods and use of the present disclosure have been described by preferred examples, those skilled in the art can apparently vary or appropriately change and combine the methods and use described herein without departing from the disclosure, spirit, and scope of the present disclosure so as to implement and use the technology of the present disclosure.

The first objective of the present disclosure is to provide a 13C-methacetin granular formulation.

The second objective of the present disclosure is to provide a method for preparing a 13C-methacetin granular formulation, which involves simple and convenient operations and achieves a high yield.

The third objective of the present disclosure is to provide use of the 13C-methacetin granular formulation in the preparation of drugs, reagents, or kits for diagnosis of a disease, evaluation of hepatic functional reserve, and preoperative and postoperative evaluation of the liver.

The disease described in the present disclosure includes one or more of liver cirrhosis, primary biliary cirrhosis, simple steatosis, a stage of chronic liver disease, alcoholic liver disease, a grade or stage of liver fibrosis, non-alcoholic fatty liver disease, neonatal cholestasis, biliary atresia, intrahepatic inflammation, acute liver failure, and hepatocellular carcinoma.

The evaluation of hepatic functional reserve described in the present disclosure includes one or more of assessment of liver function for a related disease, evaluation of liver injury from chemotherapy, prediction of death from chronic liver failure, and evaluation of liver detoxification capability.

The preoperative and postoperative evaluation described in the present disclosure includes one or more of evaluation of liver regeneration after liver resection, evaluation of the urgency of liver transplantation, evaluation of postoperative liver failure, and related preoperative and postoperative evaluation.

Here, the cosolvent is one or two selected from Tween 80, β-cyclodextrin, sodium hydroxide, sodium citrate, disodium hydrogen phosphate, sodium dihydrogen phosphate, polyethylene glycol 6000, polyethylene glycol 4000, polyethylene glycol 2000, and polyethylene glycol 1500, the wetting agent is an aqueous solution of ethanol, and they are present at a volume ratio of 1:5 to 5:1.

In a preferred embodiment of the present disclosure, the combination of the cosolvent(s) includes polyethylene glycol 4000, or polyethylene glycol 4000 and sodium dihydrogen phosphate, or polyethylene glycol 4000 and disodium hydrogen phosphate. The cosolvent is added at a ratio of 1: (0.1 to 100).

In a preferred embodiment of the present disclosure, the ratio of the mixed powder of 13C-methacetin to the wetting agent is 100 g: (1 to 100) mL, preferably 100 g: (2 to 20) mL, more preferably 100 g: (5 to 10) mL.

The ratio of the amounts of the adjuvant materials described above can ensure sufficient dissolution of 13C-methacetin granules, while the water solubility of the 13C-methacetin granular formulation can be enhanced and the stability of the granules can be improved by adding a coordinated amount of the cosolvent.

The present disclosure further provides a method for preparing the 13C-methacetin granular formulation, comprising the following steps.
1. Pulverization of Raw and Adjuvant Materials: carbon[13C]-methacetin, mannitol, and a cosolvent are respectively pulverized by using a pulverizer and sieved, and re-screened and re-checked through a 65-mesh sieve.
2. Mixing of Ingredients: the carbon[13C]-methacetin acting as a raw drug and the adjuvant materials are respectively weighed in proportion. The raw and adjuvant materials are added to a mixer and mixed.
3. Granulation Process:
   Preparation of Aqueous Solution of Ethanol: it is prepared from pharmaceutical-grade ethanol with a purity of 95% and pure water.

The mixed powder is introduced into a wet-mixing granulator, a wetting agent is proportionally added to the mixture, and at the same time stirring is started to make a soft material.

The soft material is placed and granulated in a rocking granulator, and the resulting granules with a uniform size are placed in a stainless-steel container.

4. Drying Process: the wet granules are put into a vacuum drying oven and dried at 45 °C for 3 hours, and the dried granules are sieved to obtain target granules.

5. Overall Mixing and Packaging: the granules are mixed in a mixer until the granules are uniform, and packaging of the overall-mixed granules is completed.

In a preferred embodiment of the present disclosure, the drying is performed at a temperature of 30 to 100 °C, preferably 45°C. Further, the drying method includes forced air drying and vacuum drying, preferably vacuum drying.

For example, in different embodiments, the drying temperature may be adjusted and selected according to actual needs, and the drying time may be appropriately shortened or extended depending on the type and amount of the solvent.

In a preferred embodiment of the present disclosure, the drying is performed for a period of 1 to 8 h.

The present disclosure further provides use of the 13C-methacetin granular formulation described above in the preparation of drugs, reagents, or kits for diagnosis of a disease, evaluation of hepatic functional reserve, and preoperative and postoperative evaluation of the liver.

The disease includes one or more of liver cirrhosis, primary biliary cirrhosis, simple steatosis, a stage of chronic liver disease, alcoholic liver disease, a grade or stage of liver fibrosis, non-alcoholic fatty liver disease, neonatal cholestasis, biliary atresia, intrahepatic inflammation, acute liver failure, and hepatocellular carcinoma.

The evaluation of hepatic functional reserve includes one or more of assessment of liver function for a related disease, evaluation of liver injury from chemotherapy, prediction of death from chronic liver failure, and evaluation of liver detoxification capability.

The preoperative and postoperative evaluation includes one or more of evaluation of liver regeneration after liver resection, evaluation of the urgency of liver transplantation, evaluation of postoperative liver failure, and related preoperative and postoperative evaluation.

The present invent further provides a method for diagnosis of a disease, a method for evaluation of hepatic functional reserve, or a method for preoperative and postoperative evaluation of the liver, comprising: administering the 13C-methacetin granular formulation described above or a 13C-methacetin granular formulation prepared by the preparation method described above. The disease includes one or more of liver cirrhosis, primary biliary cirrhosis, simple steatosis, a stage of chronic liver disease, alcoholic liver disease, a grade or stage of liver fibrosis, non-alcoholic fatty liver disease, neonatal cholestasis, biliary atresia, intrahepatic inflammation, acute liver failure, and hepatocellular carcinoma; the evaluation of hepatic functional reserve includes one or more of assessment of liver function for a related disease, evaluation of liver injury from chemotherapy, prediction of death from chronic liver failure, and evaluation of liver detoxification capability; and the preoperative and postoperative evaluation includes one or more of evaluation of liver regeneration after liver resection, evaluation of the urgency of liver transplantation, evaluation of postoperative liver failure, and related preoperative and postoperative evaluation.

In a preferred embodiment of the present disclosure, the pharmaceutical granular formulation includes orally administered granules, capsule granules, and the like.

In the 13C-methacetin granular formulation, the preparation method therefor and use thereof according to the present disclosure, the raw materials and reagents used are all commercially available.

The present disclosure is further described below with reference to examples.

### Example 1

This example provided a method for preparing a 13C-methacetin granular formulation, which was carried out by the following steps:
weighing 1 g of a crude 13C-methacetin product, 20 g of mannitol, and 5 g of polyethylene glycol 4000 and pulverizing, mixing, granulating, and drying the above materials step by step, whereby 25.1 g of 13C-methacetin granules were obtained, with a yield of 96.5%, a content of 100.4%, and a purity of 99.9%.

### Example 2

This example provided a method for preparing a 13C-methacetin granular formulation, comprising the steps of:
weighing 1 g of a crude 13C-methacetin product, 50 g of mannitol, 5 g of polyethylene glycol 4000, and 0.5 g of sodium dihydrogen phosphate, and pulverizing, mixing, granulating, and drying the above materials step by step, thereby obtaining 53.3 g of 13C-methacetin granules, with a yield of 94.3%, a content of 99.8%, and a purity of 99.9%.

### Example 3

This example provided a method for preparing a 13C-methacetin granular formulation, comprising the steps of:
weighing 1 g of a crude 13C-methacetin product, 60 g of mannitol, 3 g of polyethylene glycol 4000, and 1 g of disodium hydrogen phosphate, and pulverizing, mixing, granulating, and drying the above materials step by step, thereby obtaining 62.5 g of 13C-methacetin granules, with a yield of 96.2%, a content of 100.1 %, and a purity of 100%.

### Example 4

This example provided a method for preparing a 13C-methacetin granular formulation, comprising the steps of:
weighing 1 g of a crude 13C-methacetin product, 50 g of mannitol, 3 g of polyethylene glycol 2000, and 2 g of disodium hydrogen phosphate, and pulverizing, mixing, granulating, and drying the above materials step by step, thereby obtaining 53.1 g of 13C-methacetin granules, with a yield of 96.2%, a content of 99.8%, and a purity of 100%.

### Example 5

This example provided a method for preparing a 13C-methacetin granular formulation, comprising the steps of:
weighing 1 g of a crude 13C-methacetin product, 50 g of mannitol, 3 g of Tween 80, and 1 g of sodium citrate, and pulverizing, mixing, granulating, and drying the above materials step by step, thereby obtaining 52.8 g of 13C-methacetin granules, with a yield of 96.0%, a content of 99.5%, and a purity of 99.9%.

### Example 6

1 g of a crude 13C-methacetin product, 30 g of mannitol, 3 g of β-cyclodextrin, and 1 g of disodium hydrogen phosphate were weighed and were pulverized, mixed, granulated, and dried step by step, whereby 32.7g of 13C-methacetin granules were obtained, with a yield of 93.4%, a content of 99.8%, and a purity of 100%.

### Effect Example 1

The results of long-term stability testing (25±2 °C/60%±5% RH) of the 13C-methacetin granular formulations prepared in Examples 1 to 6 of the present disclosure were shown in Tables 1 to 6.

**Table 1. Results of Stability Testing of the 13C-Methacetin Granular Formulation Prepared in Example 1**

| Examination Items | Limit Requirement | 0 Month | 3 Months | 6 Months | 9 Months | 12 Months |
|---|---|---|---|---|---|---|
| Appearance | This product appears as white granules | acceptable | acceptable | acceptable | acceptable | acceptable |
| Single Impurity | ≤ 0.1 | undetected | undetected | undetected | 0.01 | 0.01 |
| Total Impurities | ≤ 0.5 | 0.01 | undetected | 0.01 | undetected | undetected |
| Water Content (%) | < 0.5 | 0.04 | 0.03 | 0.03 | 0.05 | 002 |
| Content (%) | 98.0 to 102.0 | 100.4 | 100.2 | 99.7 | 100.2 | 100.1 |

**Table 2. Results of Stability Testing of the 13C-Methacetin Granular Formulation Prepared in Example 2**

| Examination Items | Limit Requirement | 0 Month | 3 Months | 6 Months | 9 Months | 12 Months |
|---|---|---|---|---|---|---|
| Appearance | This product appears as white granules | acceptable | acceptable | acceptable | acceptable | acceptable |
| Single Impurity | ≤ 0.1 | undetected | undetected | undetected | undetected | undetected |
| Total Impurities | ≤ 0.5 | 0.01 | undetected | undetected | 0.01 | 0.01 |
| Water Content (%) | < 0.5 | 0.04 | 0.03 | 0.04 | 0.04 | 0.03 |
| Content (%) | 98.0 to 102.0 | 99.8 | 99.6 | 100.2 | 100.4 | 99.7 |

**Table 3. Results of Stability Testing of the 13C-Methacetin Granular Formulation Prepared in Example 3**

| Examination Items | Limit Requirement | 0 Month | 3 Months | 6 Months | 9 Months | 12 Months |
|---|---|---|---|---|---|---|
| Appearance | This product appears as white granules | acceptable | acceptable | acceptable | acceptable | acceptable |
| Single Impurity | ≤ 0.1 | undetected | undetected | undetected | undetected | undetected |
| Total Impurities | ≤ 0.5 | undetected | undetected | undetected | 0.01 | 0.01 |
| Water Content (%) | < 0.5 | 0.05 | 0.05 | 0.03 | 0.04 | 0.03 |
| Content (%) | 98.0 to 102.0 | 100.1 | 100.3 | 99.8 | 100.4 | 100.7 |

**Table 4. Results of Stability Testing of the 13C-Methacetin Granular Formulation Prepared in Example 4**

| Examination Items | Limit Requirement | 0 Month | 3 Months | 6 Months | 9 Months | 12 Months |
|---|---|---|---|---|---|---|
| Appearance | This product appears as white granules | acceptable | acceptable | acceptable | acceptable | acceptable |
| Single Impurity | ≤ 0.1 | undetected | undetected | undetected | undetected | undetected |
| Total Impurities | ≤ 0.5 | undetected | undetected | 0.01 | undetected | 0.01 |
| Water Content (%) | < 0.5 | 0.06 | 0.04 | 0.05 | 0.04 | 0.03 |
| Content (%) | 98.0 to 102.0 | 99.8 | 100.1 | 100.2 | 100.1 | 100.3 |

**Table 5. Results of Stability Testing of the 13C-Methacetin Granular Formulation Prepared in Example 5**

| Examination Items | Limit Requirement | 0 Month | 3 Months | 6 Months | 9 Months | 12 Months |
|---|---|---|---|---|---|---|
| Appearance | This product appears as white granules | acceptable | acceptable | acceptable | acceptable | acceptable |
| Single Impurity | ≤ 0.1 | undetected | undetected | 0.01 | 0.01 | 0.01 |
| Total Impurities | ≤ 0.5 | 0.01 | 0.01 | undetected | undetected | 0.01 |
| Water Content (%) | < 0.5 | 0.06 | 0.07 | 0.06 | 0.05 | 0.05 |
| Content (%) | 98.0 to 102.0 | 99.5 | 100.2 | 100.2 | 99.7 | 100.4 |

**Table 6. Results of Stability Testing of the 13C-Methacetin Granular Formulation Prepared in Example 6**

| Examination Items | Limit Requirement | 0 Month | 3 Months | 6 Months | 9 Months | 12 Months |
|---|---|---|---|---|---|---|
| Appearance | This product appears as white granules | acceptable | acceptable | acceptable | acceptable | acceptable |
| Single Impurity | ≤ 0.1 | undetected | undetected | undetected | 0.01 | 0.01 |
| Total Impurities | ≤ 0.5 | undetected | undetected | undetected | undetected | 0.01 |
| Water Content (%) | < 0.5 | 0.03 | 0.04 | 0.03 | 0.04 | 0.03 |
| Content (%) | 98.0 to 102.0 | 99.8 | 99.7 | 100.2 | 100.4 | 100.7 |

### Effect Example 2

### Experimental Groups 1 to 6: the 13C-methacetin granular formulations prepared in Examples 1 to 6 of the present disclosure

Control Group 1: a powdered formulation. The respective adjuvant materials and the raw drug were weighed at a prescribed ratio and mixed uniformly in a mixer for 3 hours, whereby a powdered formulation or a powder was prepared.

Control Group 2: another granular formulation. 1 g of a crude 13C-methacetin product and 25 g of mannitol were weighed and were pulverized, mixed, granulated, and dried step by step, whereby 25.1 g of 13C-methacetin granules were obtained, with a yield of 96.5%, a content of 100.4%, and a purity of 99.9%.

The comparison results are shown in Table 7.

**Table 7. Comparison of Water Solubility and Stability**

| Detected Items | | Solubility | Stability: Appearance | Stability: Impurity A | Stability: Impurity B |
|---|---|---|---|---|---|
| Experimental Group 1 | the granular formulation prepared in Example 1 | 75 mg/100 ml, dissolved quickly | white powder did not agglomerate | 0.01% | undetected |
| Experimental Group 2 | the granular formulation prepared in Example 2 | 75 mg/100 ml, dissolved quickly | white powder did not agglomerate | undetected | undetected |
| Experimental Group 3 | the granular formulation prepared in Example 3 | 75 mg/100 ml, dissolved quickly | white powder did not agglomerate | undetected | undetected |
| Experimental Group 4 | the granular formulation prepared in Example 4 | 75 mg/100 ml, dissolved quickly | white powder did not agglomerate | undetected | undetected |
| Experimental Group 5 | the granular formulation prepared in Example 5 | 75 mg/100 ml, dissolved quickly | white powder did not agglomerate | 0.01% | undetected |
| Experimental | the granular formulation | 75 mg/100 ml, | white powder | 0.01% | undetected |
| Group 6 | prepared in Example 6 | dissolved quickly | did not agglomerate | | |
| Control Group 1 | a powdered formulation | 60 mg/100 ml, dissolved slowly | white powder agglomerated | 0.15% | undetected |
| Control Group 2 | a granular formulation | 50 mg/100 ml, dissolved slowly | white powder agglomerated | 0.63% | 0.19% |

13C-methacetin has a low solubility in water, has a slow dissolution rate, should be stirred for a long time when being dissolved in 100 ml of water in an amount of 50 mg, and thus is not convenient in oral administration. During storage, the powder is likely to agglomerate and undergo a degradation, which affects the quality of the product. The powdered formulation is a mixed powder, and the water solubility of the product is improved after adjuvant materials are added thereto, but it still cannot meet the requirements of clinical use, and its dissolution rate is also slow. The powders are agglomerated during storage. The addition of the adjuvant materials helps to improve the stability of the product, and the degraded impurities are less than those of the raw drug powder. The developed granules, which are prepared by modifying the prescription and developing formulation processes, exhibits a water solubility meeting the clinical requirements and does not agglomerate during storage, and has a very small amount of an impurity that is far below the standard limit because the degradation is effectively inhibited. By comparison, the developed granular formulation has better stability and water solubility, is stable in quality, and has high value in use.

The 13C-methacetin granular formulation and the preparation method therefor and use thereof according to the present disclosure have been described in detail above. The principles and embodiments of the present disclosure are described herein by using specific examples, and the above examples are described only to help understand the method and the core concept of the present disclosure. It should be noted that, as appreciated by those skilled in the art, several improvements and modifications can also be made to the present disclosure without departing from the principle of the present disclosure, and these improvements and modifications also fall within the scope of protection of the claims of the present disclosure.

## Claims

1. A 13C-methacetin granular formulation, comprising 13C-methacetin, mannitol, a cosolvent, and a wetting agent,
wherein the cosolvent comprises one or more of Tween 80, β-cyclodextrin, sodium hydroxide, sodium citrate, disodium hydrogen phosphate, sodium dihydrogen phosphate, polyethylene glycol 6000, polyethylene glycol 4000, polyethylene glycol 2000, and polyethylene glycol 1500; and
the wetting agent comprises one or both of water or an aqueous solution of ethanol.

2. The 13C-methacetin granular formulation according to claim 1, wherein a weight ratio of the 13C-methacetin to the cosolvent is 1: (0.1 to 100).

3. The 13C-methacetin granular formulation according to claim 1 or 2, wherein a weight ratio of a mixture of the 13C-methacetin, mannitol, and the cosolvent to the wetting agent is 100: (1 to 100), calculated as g/mL.

4. The 13C-methacetin granular formulation according to claim 3, wherein a weight ratio of a mixture of the 13C-methacetin and the cosolvent to the wetting agent is 100: (1 to 20), calculated as g/mL; and the wetting agent is the aqueous solution of ethanol, and a volume ratio of ethanol to water in the aqueous solution of ethanol is 1:5 to 5:1.

5. The 13C-methacetin granular formulation according to any one of claims 1 to 4, wherein a weight ratio of the 13C-methacetin to mannitol is 1: (20 to 60).

6. A method for preparing the 13C-methacetin granular formulation according to any one of claims 1 to 5, wherein the 13C-methacetin is pulverized and then mixed with mannitol and the cosolvent to obtain a mixture, and the mixture is mixed with the wetting agent and then granulated and dried to obtain the 13C-methacetin granular formulation.

7. The method according to claim 6, wherein the drying is performed at a temperature of 30 to 100 °C, and the drying is performed for a period of 1 to 8 h.

8. The method according to claim 7, wherein the drying is performed at a temperature of 45 °C, and the drying is performed for a period of 3 h.

9. Use of the 13C-methacetin granular formulation according to any one of claims 1 to 5 or the 13C-methacetin granular formulation prepared by the method according to any one of claims 6 to 8 in a preparation of drugs, reagents, or kits for diagnosis of a disease, an evaluation of hepatic functional reserve, and a preoperative and postoperative evaluation of liver.

10. The use according to claim 9, wherein the disease comprises one or more of liver cirrhosis, primary biliary cirrhosis, simple steatosis, a stage of chronic liver disease, alcoholic liver disease, a grade or stage of liver fibrosis, non-alcoholic fatty liver disease, neonatal cholestasis, biliary atresia, intrahepatic inflammation, acute liver failure, and hepatocellular carcinoma;
the evaluation of hepatic functional reserve comprises one or more of an assessment of liver function for a related disease, an evaluation of liver injury from chemotherapy, a prediction of death from chronic liver failure, and an evaluation of liver detoxification capability; and
the preoperative and postoperative evaluation comprises one or more of an evaluation of liver regeneration after liver resection, an evaluation of urgency of liver transplantation, an evaluation of postoperative liver failure, and a related preoperative and postoperative evaluation.

11. A method for diagnosis of a disease, a method for evaluation of hepatic functional reserve, or a method for preoperative and postoperative evaluation of liver, comprising: administering the 13C-methacetin granular formulation according to any one of claims 1 to 5 or the 13C-methacetin granular formulation prepared by the method according to any one of claims 6 to 8.

12. The method for diagnosis of a disease, the method for evaluation of hepatic functional reserve, or the method for preoperative and postoperative evaluation of liver according to claim 11, wherein the disease comprises, but is not limited to, one or more of liver cirrhosis, primary biliary cirrhosis, simple steatosis, a stage of chronic liver disease, alcoholic liver disease, a grade or stage of liver fibrosis, non-alcoholic fatty liver disease, neonatal cholestasis, biliary atresia, intrahepatic inflammation, acute liver failure, and hepatocellular carcinoma;
the evaluation of hepatic functional reserve comprises, but is not limited to, one or more of an assessment of liver function for a related disease, an evaluation of liver injury from chemotherapy, a prediction of death from chronic liver failure, and an evaluation of liver detoxification capability; and
the preoperative and postoperative evaluation comprises, but is not limited to, one or more of an evaluation of liver regeneration after liver resection, an evaluation of urgency of liver transplantation, an evaluation of postoperative liver failure, and a related preoperative and postoperative evaluation.
